# FASCICULE DE BREVET EUROPEEN

(11) **EP 0 707 862 B1**
(45) Date de publication et mention de la délivrance du brevet: **27.06.2001**
(21) Numéro de dépôt: 95402283.6
(22) Date de dépôt: 13.10.1995
(51) Int. Cl.: A61M 15/00

(54) **Dispositif pour l'inhalation de produits en poudre**
Inhaliervorrichtung für pulverförmige Substanzen
Inhalation device for powdery substances

(30) Priorité: 18.10.1994 FR 9412399
(43) Date de publication de la demande: 24.04.1996
(73) Titulaire: REXAM SOFAB, 76450 Le Tréport (FR)
(72) Inventeur: Bougamont, Jean-Louis, F-76260 Eu (FR); Leuliet, David, F-80350 Mers-Les-Bains (FR); Lompech, Hervé, F-76117 Incheville (FR)
(74) Mandataire: Busnel, Jean-Benoît

(56) Documents cités:
- EP-A- 0 028 162
- EP-A- 0 406 893
- WO-A-91/02558
- FR-A- 2 662 936

## Description

La présente invention concerne un dispositif pour l'inhalation de produits en poudre et, plus particulièrement, de produits pulvérulents conditionnés en gélules.

Divers produits, spécialement des médicaments, demandent à être mis en oeuvre et, en particulier, inhalés sous forme de poudres fines. Une difficulté est alors de concilier simplicité du dispositif, efficacité, en particulier précision et dispersion de la dose délivrée, sureté, en particulier hygiène et aseptie, etc. Il apparait donc intéressant a priori de conditionner ces produits en gélules uni doses qu'il conviendra d'ouvrir, par exemple en les découpant ou en les perforant avant d'entraîner leur contenu dans le courant d'air inhalé. Cette opération délicate a déjà été effectuée selon diverses et nombreuses manières : ainsi, pour expulser le produit, il a été proposé d'opérer par gravité ou centrifugation, mise en vibration ou en dépression, balayage direct par le courant principal ou le courant induit d'un venturi.

Un dispositif selon le préambule de la revendication 1 est connu de EP-A-406893.

Mais les dispositifs antérieurs ne permettent pas de synchroniser rapidement la création ou l'émission du flux porteur de poudre avec l'inhalation. De plus, ces dispositifs doivent être rechargés fréquemment, ce qui rend leur utilisation laborieuse.

La présente invention a pour but de résoudre ces problèmes techniques de manière satisfaisante.

Ce but est atteint selon l'invention au moyen d'un dispositif pour l'inhalation de produits en poudres conditionnés en gélules du type comprenant un boîtier obturé par un couvercle et renfermant des moyens de support et de transfert des gélules, des moyens d'ouverture desdites gélules, un embout de prise et un orifice d'admission d'air, dont lesdits moyens de support et de transfert des gélules sont constitués d'un chargeur mobile, pourvu d'au moins deux alvéoles destinées à recevoir chacune une gélule et de moyens anti-retour permettant d'obtenir par entraînement dû couvercle un déplacement pas à pas, à sens unique, du chargeur dans le boîtier et d'immobiliser successivement chaque alvéole dans une position d'inhalation où sa gélule communique avec l'embout de prise et dont lesdits moyens d'ouverture sont constitués de deux lames parallèles respectivement solidaires du boîtier et du couvercle adaptées pour couper chacune une des extrémités longitudinales d'une gélule par déplacements successifs relativement au boîtier d'abord du couvercle entraînant le chargeur dans un premier sens puis du couvercle seul en sens inverse après verrouillage du chargeur en position d'inhalation.

Selon un mode de réalisation avantageux, ledit chargeur mobile est un barillet rotatif autour d'un axe central et lesdits moyens anti-retour sont constitués d'au moins deux cliquets destinés à coopérer avec des taquets ménagés sur le pourtour du boîtier et/ou du couvercle.

De préférence, le couvercle du boîtier est cylindrique et a un axe de rotation confondu avec l'axe central du chargeur.

Dans ce cas, l'extrémité libre des pattes des cliquets est située sur le rayon du barillet passant par le centre d'une alvéole.

Selon une caractéristique avantageuse de l'invention, les lames du boîtier et du couvercle sont disposées de part et d'autre des alvéoles et comportent un orifice central dont le diamètre est légèrement supérieur à celui des gélules et qui communique avec l'embout de prise et l'orifice d'admission d'air en position d'inhalation.

L'entraînement du chargeur par le couvercle s'effectue de préférence au moyen d'un système à roue libre.

De plus, ledit couvercle comporte au moins un tenon périphérique engagé de manière coulissante dans une rainure interrompue ménagée sur le pourtour du boîtier et contre les extrémités de laquelle le tenon vient en butée pour limiter la course dudit couvercle par rapport audit boîtier.

La longueur de la rainure périphérique du boîtier correspond à la distance séparant deux alvéoles du chargeur.

Par ailleurs, il est prévu que les cliquets du chargeur sont constitués de pattes s'étendant de façon sensiblement tangentielle à la périphérie du chargeur et qui sont susceptibles de fléchir élastiquement au contact des taquets pour s'escamoter.

Selon une autre caractéristique, l'embout de prise comporte un conduit interne d'échappement divergent et obturable par un clapet tandis que ledit orifice d'admission d'air est ménagé au travers du couvercle et est pourvu d'une grille protectrice.

Selon une première variante de réalisation, le boîtier comporte une coupelle dans laquelle est logé le chargeur.

Lorsque le chargeur est un barillet rotatif, l'axe de ladite coupelle coïncide avec l'axe central de rotation du barillet et est décalé par rapport à l'axe de l'embout de prise.

Eventuellement, le boîtier comportera une lumière disposée en regard de l'une des positions d'arrêt des alvéoles du chargeur pour former un voyant de contrôle du remplissage dudit chargeur.

Selon encore une autre caractéristique, la longueur des alvéoles du chargeur est inférieure à la longueur des gélules et les bords tranchants des lames du boîtier et du couvercle sont disposés de façon à venir découper les gélules tangentiellement à leur paroi latérale cylindrique.

Selon une autre variante de réalisation, le chargeur est un barillet qui comporte sept alvéoles disposées selon un cercle et sept cliquets périphériques correspondants.

La présente invention fournit un dispositif de poche facile à utiliser car interdisant un double dosage et n'exigeant pour l'inhalation, aucune coordination précise de l'inspiration avec une commande manuelle tout en protégeant l'utilisateur contre une expiration accidentelle.

Le boîtier est muni d'un embout de prise tel qu'un embout d'aspiration buccal et porte le mécanisme permettant la distribution du produit.

Ce mécanisme comprend des moyens de support et de transfert amenant tour à tour les gélules en position d'inhalation par un mouvement pas à pas grâce à des organes d'arrêt à support élastique déformable pourvus de moyens anti-retour pour les y immobiliser en regard d'un orifice d'échappement vers le canal interne de l'embout précité ; il s'agira en principe d'un chargeur mobile par exemple tournant à la manière d'un barillet ou susceptible d'être déplacé de façon linéaire et guidée dans un logement rectiligne. Les gélules y seront placées soit individuellement soit intégrées à un conditionnement, avantageusement interchangeable, tel qu'une bande ou encore un disque formant éventuellement lui-même le chargeur.

Il comprend ensuite deux lames tranchantes dirigées dans le même sens le long de deux faces opposées du chargeur, à distance telle que leur mouvement relatif les amène à trancher les deux extrémités de toute gélule au moment de sa mise en place en position d'inhalation devant l'orifice d'échappement. Pour faciliter cette opération, les arêtes des lames tranchantes seront avantageusement obliques et leur entrée en action décalée.

Un organe d'entraînement ou entraîneur, en principe irréversible et à commande manuelle, déplacera successivement le chargeur d'une position d'arrêt à la suivante. L'emploi d'une transmission démultipliée telle que vis sans fin ou croix de Malte n'est pas nécessaire ; on lui préférera le moyen simple d'un système à roue libre en va-et-vient entre deux butées, en particulier un système à chargeur en blocage alternatif, d'abord à l'aller sur un entraîneur à bouton de manoeuvre de même axe que lui, ensuite sur le boîtier au retour de cet entraîneur, éventuellement aidé alors par un ressort : pour solidariser les deux organes, on peut imaginer ici des rochets à bille mais on préférera le simple emploi de cliquets travaillant dans le même sens sur deux voies parallèles ; le cas échéant les talons des lames tranchantes elles-mêmes pourraient remplir ce rôle.

Les deux lames seront alors de préférence montées respectivement l'une sur le boîtier l'autre sur l'entraîneur, la première agissant lors du mouvement d'avance par lequel celui-ci entraîne le chargeur, la seconde lorsqu'il est ramené, seul, à sa position de départ. Les deux chutes créées par l'ouverture de la gélule seront avantageusement évacuées vers un réceptacle interne.

L'invention sera mieux comprise à la lecture de la description qui va suivre, d'un mode de réalisation particulier, accompagnée des dessins sur lesquels :
- les figures 1a et 1b représentent des vues extérieures d'ensemble du dispositif de l'invention respectivement assemblé et démonté ;
- la figure 2 représente une vue en coupe longitudinale selon la ligne I-I du dispositif de l'invention ;
- les figures 3a et 3b représentent des vues en coupe transversale selon III-III du dispositif de la figure 2 respectivement avec et sans le chargeur ; et,
- les figures 4a et 4b représentent des vues en coupe transversale selon IV-IV du dispositif de la figure 2 respectivement avec et sans le chargeur.

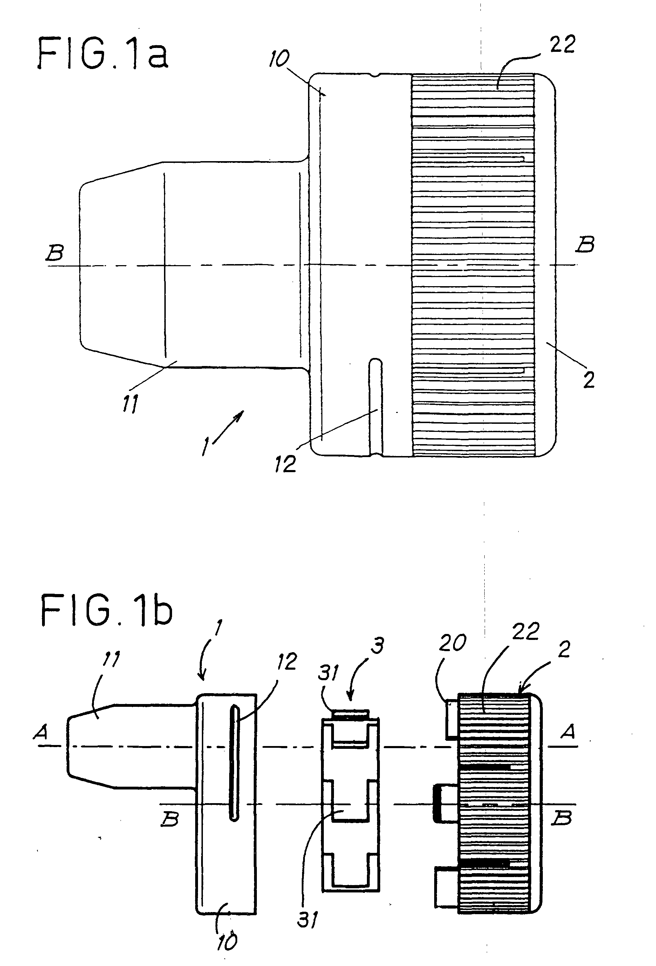

Le dispositif d'inhalation représenté sur les figures 1a et 1b comprend un boîtier 1 constitué notamment d'une coupelle circulaire 10 d'axe B portant un embout de prise 11 d'axe A et un couvercle 2 cylindrique venant obturer le boîtier 1 en s'emboîtant tournant autour de l'axe B sur la coupelle 10. L'embout de prise 11 comporte un conduit interne d'échappement 11a divergent et obturable par un clapet pivotant 5.

Le couvercle 2 comporte au moins un tenon périphérique 20 de retenue engagé de manière coulissante dans une rainure interrompue 12 ménagée sur le pourtour de la coupelle 10 du boîtier 1. Le tenon 20 vient en butée contre les extrémités de la rainure 12 pour limiter la course en rotation du couvercle 2 par rapport au boîtier 1.

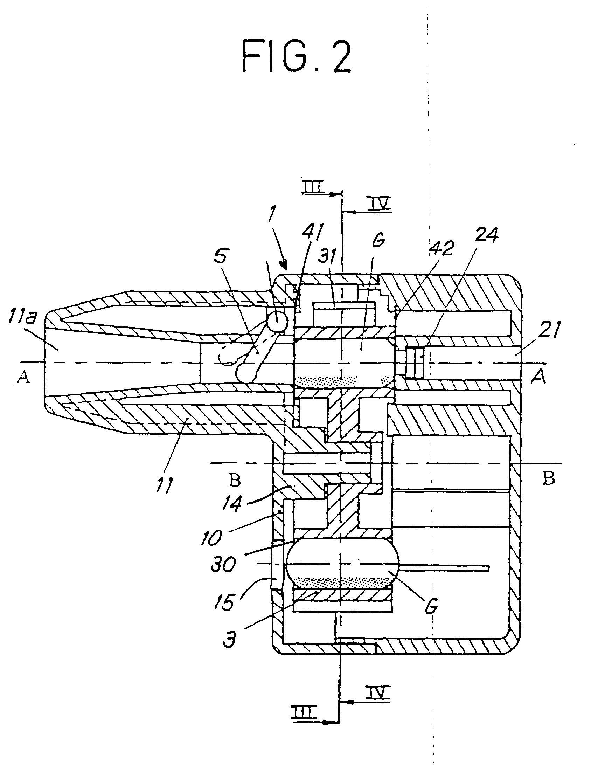

Le couvercle 2 comporte en outre un orifice d'admission d'air 21 (voir figure 2) situé, en position d'inhalation, dans le prolongement de l'axe A de l'embout de prise 11.

L'orifice d'admission d'air est équipé d'une grille protectrice 24.

La face latérale du couvercle 2 est munie de cannelures 22 facilitant la prise.

Le boîtier 1 et le couvercle 2 renferment des moyens de support et de transfert des gélules de produit pulvérulent.

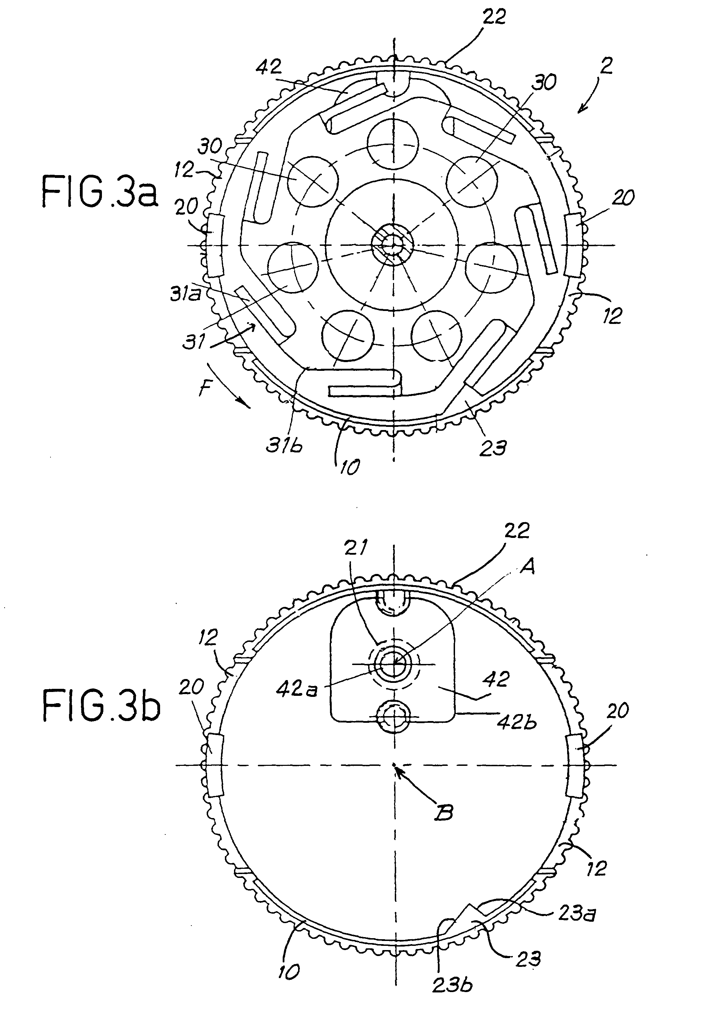

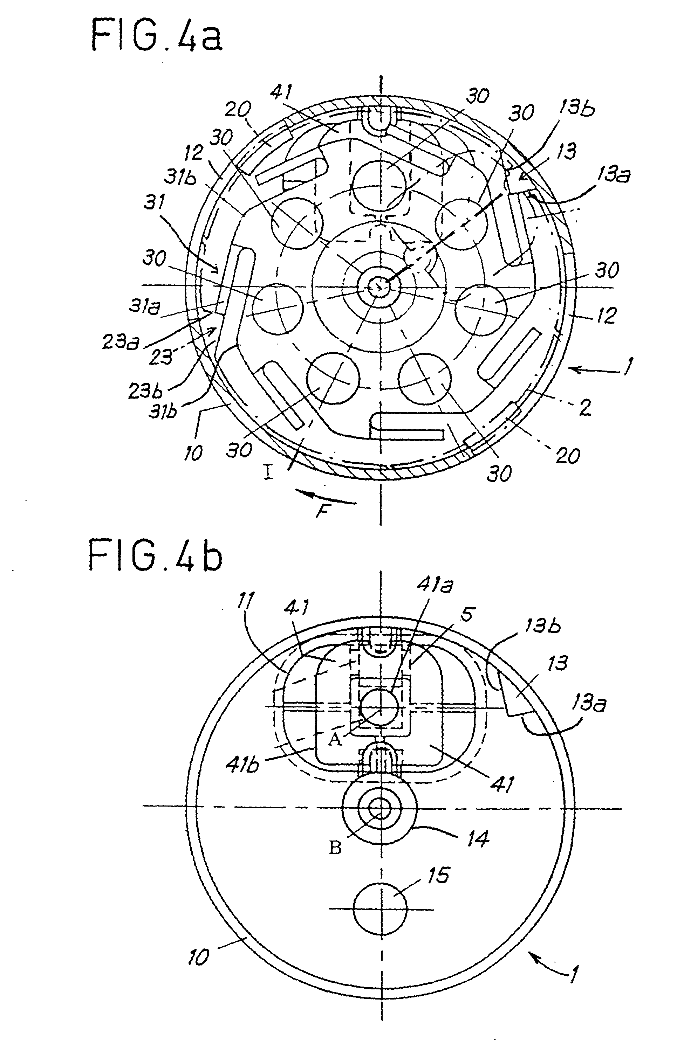

Dans le mode de réalisation des figures 1b et 2 ces moyens sont constitués d'un chargeur 3 sous forme d'un barillet rotatif autour de l'axe central B qui est logé dans la coupelle 10 et est pourvu d'au moins deux alvéoles 30 destinées à recevoir chacune une gélule G contenant une dose de médicament. La longueur des alvéoles 30 est légèrement inférieure à la longueur des gélules. Dans le mode de réalisation des figures 3a et 4a, le barillet comporte sept alvéoles 30 disposées selon un arc de cercle et est monté sur un moyeu 14 réalisé sur la face interne de la coupelle 10.

Le barillet 3 comporte, en outre, au moins deux cliquets périphériques 31 et, comme représenté sur les figures 3a et 4a, de préférence sept cliquets 31 correspondant aux alvéoles 30.

Les cliquets 31 sont destinés à coopérer avec des taquets 13,23 ménagés sur le pourtour du boîtier 1 et/ou du couvercle 2 pour interdire toute rotation du barillet 3 dans un sens contraire à celui de la flèche F en formant ainsi un système anti-retour.

En d'autres termes, la coopération entre les cliquets 31 du barillet 3 et les taquets 13,23 permet, par entraînement du couvercle 2 en rotation, un déplacement angulaire, pas à pas et à sens unique, du barillet 3 dans le boîtier 1. Le barillet 3 et le couvercle 2 sont reliés de manière fonctionnelle par un système d'entraînement à roue libre autorisant un retour en arrière par rapport à la flèche F, vers sa position initiale du couvercle 2 après verrouillage du barillet 3. Cette disposition permet ainsi de ramener l'orifice d'admission d'air 21 dans le prolongement de l'axe A de l'embout de prise 11 pour l'inhalation.

Ce déplacement angulaire définit autant de positions d'arrêt du barillet 3 qu'il y a de cliquets 31 et conduit à immobiliser successivement chaque alvéole 30 avec sa gélule G, longitudinalement en position d'inhalation, c'est-à-dire dans l'axe A de l'embout de prise 11.

Le barillet 3 est verrouillé en position d'inhalation grâce aux profils spécifiques des cliquets 31 et des taquets 13,23.

En effet, les cliquets 31 sont constitués de pattes 31a s'étendant de façon sensiblement tangentielle à la périphérie du barillet 3 et qui sont susceptibles de fléchir élastiquement vers l'axe central B, au contact des taquets 13,23 pour s'escamoter. Les pattes 31a se prolongent vers l'avant, dans le sens de la flèche F, par des bords coudés 31b. L'extrémité libre des pattes 31a est située sur le rayon du barillet 3 passant par le centre de l'alvéole 30 correspondante.

Les taquets 13,23 sont constitués d'ergots de section triangulaire faisant saillie en direction de l'axe B.

La face latérale avant 13a,23a des taquets 13,23 dans le sens de l'avance F forme une butée d'arrêt pour l'extrémité libre de la patte 31a tandis que leur face latérale arrière 13b,23b vient en contact d'appui, lors de la rotation du barillet 3, d'abord avec les bords coudés 31b des cliquets 31 puis avec les pattes 31a. La jonction entre la face avant 13a,23a et la face arrière 13b,23b des taquets forme un coude d'environ 90°.

La rotation du couvercle 2 entraînant le barillet 3 provoque ainsi une déformation élastique, croissante et continue, des pattes 31a au contact de la face arrière 13,23b des taquets 13,23 par effet de came.

La poursuite de la rotation entraîne l'escamotage des pattes 31a, au franchissement de la jonction coudée entre les faces avant 13a,23a et arrière 13b,23b des taquets et leur encliquetage (position en traits mixtes sur figure 4a).

Les taquets 13,23 sont diamétralement décalés pour toujours coopérer avec deux cliquets 31 différents. La face arrière 13b,23b des taquets s'étend dans le prolongement rectiligne des pattes 31a lorsque celles-ci sont en butée contre la face avant 13a,23a des taquets.

Il subsiste cependant un léger jeu permettant un débattement angulaire sans déformation autour de chaque position d'arrêt du barillet 3.

La longueur de la rainure 12 de la coupelle 10 du boîtier dans laquelle coulisse le tenon 20 de retenue du couvercle 2, correspond donc sensiblement à la distance angulaire séparant deux alvéoles 30 ou deux pattes 31a du barillet 3. Dans le mode de réalisation des figures, la course du tenon correspond à un déplacement angulaire d'environ 60°.

Le boîtier 1 et le couvercle 2 renferment également des moyens d'ouverture des gélules G.

Dans le mode de réalisation représenté, ces moyens sont constitués de deux lames parallèles 41,42 respectivement solidaires du boîtier 1 et du couvercle 2.

Les lames 41,42 sont adaptées pour couper chacune, une des extrémités longitudinales d'une gélule G par rotations successives relativement au boîtier 1 d'abord dans un premier sens F, du couvercle 2 entraînant le barillet 3 puis du couvercle 2 seul, en sens inverse après verrouillage dudit barillet en position d'inhalation. Les lames 41,42 sont, de préférence identiques et disposées symétriquement de part et d'autre des alvéoles 30. Elles comportent un orifice central 41a,42a dont le diamètre est légèrement supérieur à celui des gélules G pour permettre l'évacuation du produit.

A cet effet, les axes respectifs des orifices 41a,42a des lames 41,42 sont susceptibles de coïncider entre eux et avec l'axe commun A de l'embout de prise 11 et de l'orifice d'admission d'air 21, en position d'inhalation. Les bords tranchants 41b,42b des lames 41,42 sont disposés de façon à venir découper tout à tour les gélules G tangentiellement à leur paroi latérale cylindrique, au ras du bord des alvéoles.

Les chutes tombent dans les espaces libres existant derrière les lames 41,42.

Une voie se trouve ainsi ouverte entre l'orifice d'admission 21 et le conduit d'échappement 11a à l'entrée de l'embout 11 qui va permettre à l'air de balayer directement la poudre lorsque le patient aspirera par l'embout buccal.

Avantageusement, la coupelle 10 du boîtier 1 comporte sur sa face avant, une lumière 15 formant voyant disposée en regard de l'une des positions d'arrêt des alvéoles 30 du barillet 3 pour contrôler son niveau de remplissage.

## Revendications

1. Dispositif pour l'inhalation de produits en poudres conditionnés en gélules (G) du type comprenant un boîtier (1) obturé par un couvercle (2) et renfermant des moyens de support et de transfert des gélules (G), des moyens d'ouverture à deux lames desdites gélules (G), un embout de prise (11) et un orifice d'admission d'air (21), dont
lesdits moyens de support et de transfert des gélules sont constitués d'un chargeur mobile (3), pourvu d'au moins deux alvéoles (30) destinées à recevoir chacune une gélule (G) et de moyens anti-retour permettant d'obtenir par entraînement du couvercle (2), un déplacement pas à pas, à sens unique, du chargeur (3) dans le boîtier (1) et caractérisé en ce que ledit chargeur (3) et les moyens anti-retour permettent d'immobiliser successivement chaque alvéole (30) dans une position d'inhalation où sa gélule (G) communique avec l'embout de prise (11) et en ce que lesdits moyens d'ouverture sont constitués de deux lames parallèles (41,42) respectivement solidaires du boîtier (1) et du couvercle (2) adaptées pour couper chacune une des extrémités longitudinales d'une gélule (G) par déplacements successifs, relativement au boîtier (1), d'abord du couvercle (2) entraînant le chargeur (3) dans un premier sens (F), puis du couvercle (2) seul, en sens inverse, après verrouillage du chargeur (3) en position d'inhalation.

2. Dispositif selon la revendication 1, caractérisé en ce que ledit chargeur mobile (3) est un barillet rotatif autour d'un axe central (B).

3. Dispositif selon la revendication 1 ou 2, caractérisé en ce que lesdits moyens anti-retour sont constitués d'au moins deux cliquets (31) destinés à coopérer avec des taquets (13,23) ménagés sur le pourtour du boîtier (1) et/ou du couvercle (2).

4. Dispositif selon l'une des revendications précédentes,
caractérisé en ce que les lames (41,42) du boîtier (1) et du couvercle (2) sont disposées de part et d'autre des alvéoles (30) et comportent un orifice central (41a,42a) dont le diamètre est légèrement supérieur à celui des gélules (G) et qui communique avec l'embout de prise (11) et l'orifice d'admission d'air (21) en position d'inhalation.

5. Dispositif selon la revendication 2,
caractérisé en ce que le couvercle (2) du boîtier (1) est cylindrique et a un axe de rotation confondu avec l'axe central (B) du chargeur (3).

6. Dispositif selon l'une des revendications précédentes,
caractérisé en ce que le chargeur (3) et le couvercle (2) sont reliés par un système d'entraînement à roue libre.

7. Dispositif selon l'une des revendications précédentes, caractérisé en ce que ledit couvercle (2) comporte au moins un tenon périphérique (20) engagé de manière coulissante dans une rainure interrompue (12) ménagée sur le pourtour du boîtier (1) et contre les extrémités de laquelle le tenon (20) vient en butée pour limiter la course dudit couvercle (2) par rapport audit boîtier (1).

8. Dispositif selon l'une des revendications précédentes, caractérisé en ce que l'embout de prise (11) comporte un conduit interne d'échappement (11a) divergent et obturable par un clapet (5).

9. Dispositif selon la revendication 3, caractérisé en ce que lesdits cliquets (31) du chargeur (3) sont constitués de pattes (31a) s'étendant de façon sensiblement tangentielle à la périphérie du chargeur (3) et qui sont susceptibles de fléchir élastiquement au contact des taquets (13,23) pour s'escamoter.

10. Dispositif selon les revendications 3 et 9,
caractérisé en ce que l'extrémité libre des pattes (31a) des cliquets (31) est située sur le rayon du barillet (3) passant par le centre d'une alvéole (30).

11. Dispositif selon la revendication 7,
caractérisé en ce que la longueur de la rainure périphérique (12) du boîtier (1) correspond à la distance séparant deux alvéoles (30) du chargeur (3).

12. Dispositif selon l'une des revendications précédentes, caractérisé en ce que ledit orifice d'admission d'air (21) est ménagé au travers du couvercle (2) et est pourvu d'une grille protectrice (24).

13. Dispositif selon l'une des revendications précédentes, caractérisé en ce que le boîtier (1) comporte une coupelle (10) dans laquelle est logé le chargeur (3).

14. Dispositif selon les revendications 2 et 13, caractérisé en ce que l'axe de ladite coupelle coïncide avec l'axe central (B) de rotation du barillet (3).

15. Dispositif selon l'une des revendications précédentes, caractérisé en ce que ledit boîtier (1) comporte une lumière (15) disposée en regard de l'une des positions d'arrêt des alvéoles (30) du chargeur (3) pour former un voyant de contrôle du remplissage dudit chargeur.

16. Dispositif selon l'une des revendications précédentes, caractérisé en ce que la longueur des alvéoles (30) du chargeur (3) est inférieure à la longueur des gélules (G).

17. Dispositif selon l'une des revendications précédentes, caractérisé en ce que les bords tranchants (41b,42b) des lames (41,42) du boîtier (1) et du couvercle (2) sont disposés de façon à venir découper les gélules (G) tangentiellement à leur paroi latérale cylindrique.

18. Dispositif selon les revendications 2 et 3, caractérisé en ce que le chargeur (3) comporte sept alvéoles (30) disposées selon un cercle et sept cliquets périphériques (31) correspondants.

## Claims

1. A device for inhaling powder products packaged in capsules (G), the device being of the type comprising a housing (1) closed by a cover (2) and containing means for supporting and transferring capsules (G), opening means provided with two blades for opening said capsules (G), a dispensing endpiece (11), and an air intake orifice (21), of which said means for supporting and transferring capsules are constituted by a moving charger (3) provided with at least two chambers (30) each designed to receive a respective capsule (G), and non-return means making it possible to use the cover (2) to entrain the charger (3) inside the housing (1) in stepwise displacement in one direction only and characterised in that said charger (3) and the non-return means make it possible to lock each chamber (30) in succession in an inhalation position in which its capsule (G) is in communication with the dispensing endpiece (11), and in that said opening means are constituted by two parallel blades (41,42) respectively secured to the housing (1) and to the cover (2) and each adapted to cut off a respective one of the longitudinal ends of a capsule (G) by successive displacements of the cover (2) relative to the housing (1): firstly while entraining the charger (3) in a first direction (F); and then on its own in the opposite direction after the charger (3) has been locked in the inhalation position.

2. A device according to claim 1, characterised in that said moving charger (3) is a cylinder capable of revolving about a central axis (B).

3. A device according to claim 1 or 2, characterised in that said non-return means are constituted by at least two pawls (31) designed to co-operate with catches (13,23) formed on the periphery of the housing (1) and/or of the cover (2).

4. A device according to one of the preceding claims,
characterised in that the blades (41,42) of the housing (1) and of the cover (2) are disposed at opposite ends of the chambers (30) and include respective central orifices (41a,42a) of a diameter slightly greater than that of the capsules (G) and communicating respectively with the dispensing endpiece (11) and with the air intake orifice (21) in the inhalation position.

5. A device according to claim 2,
characterised in that the cover (2) of the housing (1) is cylindrical and has an axis of rotation that coincides with the central axis (B) of the charger (3).

6. A device according to one of the preceding claims,
characterised in that the charger (3) and the cover (2) are interconnected by a free-wheel drive system.

7. A device according to one of the preceding claims, characterised in that said cover (2) includes at least one peripheral projection (20) slidably engaged in an interrupted groove (12) formed in the periphery of the housing (1) and against the ends of which the projection (20) comes into abutment to limit the stroke of said cover (2) relative to said housing (1).

8. A device according to one of the preceding claims, characterised in that the dispensing endpiece (11) includes an internal exhaust duct (11 a) that flares and that is closeable by means of a flap (5).

9. A device according to claim 3, characterised in that said pawls (31) of the charger (3) are constituted by tabs (31a) extending substantially tangentially from the periphery of the charger (3) and which are suitable for bending elastically on contact with the catches (13,23) to pass over them.

10. A device according to claims 3 and 9,
characterised in that the free ends of the tabs (31a) of the pawls (31) are situated on radii of the cylinder (3) passing through the centers of the chambers (30).

11. A device according to claim 7,
characterised in that the length of the peripheral groove (12) of the housing (1) corresponds to the distance between two chambers (30) of the charger (3).

12. A device according to one of the preceding claims, characterised in that said air intake orifice (21) is formed through the cover (2) and is provided with a protective grid (24).

13. A device according to one of the preceding claims, characterised in that the housing (1) includes a cup (10) in which the charger (3) is received.

14. A device according to claims 2 and 13, characterised in that the axis of said cup coincides with the central axis (B) about which the cylinder (3) revolves.

15. A device according to one of the preceding claims, characterised in that said housing (1) includes an opening (15) facing one of the stop positions for the chambers (30) of the charger (3) to form a window for inspecting the filling level of said charger.

16. A device according to one of the preceding claims, characterised in that the chambers ( 30) of the charger (3) are shorter than the length of the capsules (G).

17. A device according to one of the preceding claims, characterised in that the cutting edges (41b,42b) of the blades (41,42) of the housing (1) and of the cover (2) are disposed in such a manner as to cut the capsules (G) tangentially to their cylindrical side walls.

18. A device according to claims 2 and 3, characterised in that the charger (3) has seven chambers (30) disposed on a circle, and seven corresponding peripheral pawls (31).

## Patentansprüche

1. Vorrichtung zum Inhalieren pulverförmiger Produkte, die in Gelatinekapseln (G) abgepackt sind, des Typs, der ein durch einen Deckel (2) abgeschlossenes Gehäuse (1) umfasst und Einrichtungen zum Halten und Befördern der Gelatinekapseln (G), Einrichtungen mit zwei Blättern zum Öffnen der Gelatinekapseln (G), ein Ansatzstück (11) und eine Lufteinlassöffnung (21) einschließt, wovon die Mittel zum Halten und Befördern der Gelatinekapseln von einer beweglichen Ladeeinrichtung (3) gebildet sind, die versehen ist mit mindestens zwei Zellen (30) zum Aufnehmen jeweils einer Gelatinekapsel (G) und mit Rücklaufsicherungseinrichtungen, die es ermöglichen, durch eine Mitnahme des Deckels (2) eine Schritt-für-Schritt-Bewegung in eine einzige Richtung der Ladeeinrichtung (3) im Gehäuse (1) zu erzielen, dadurch gekennzeichnet, dass die Ladeeinrichtung (3) und die Rücklaufsicherungseinrichtungen es erlauben, nacheinander jede Zelle (30) in einer Inhalationsposition zu immobilisieren, in der ihre Gelatinekapsel (G) mit dem Ansatzstück (11) in Verbindung steht, und dadurch, dass die Mittel zum Öffnen von zwei parallelen, jeweils mit dem Gehäuse (1) und dem Deckel (2) verbundenen Blättern (41, 42) gebildet sind, die jeweils dafür ausgelegt sind, eines der Längsenden einer Gelatinekapsel (G) durch aufeinander folgende Bewegungen bezüglich des Gehäuses (1) durchzuschneiden, und zwar zunächst des Deckels (2), der die Ladeeinrichtung (3) in einer ersten Richtung (F) mitnimmt, und danach des Deckels (2) alleine in der entgegengesetzten Richtung, und dies nach der Arretierung der Ladeeinrichtung (3) in der Inhalationsposition.

2. Vorrichtung nach Anspruch 1, dadurch gekennzeichnet, dass die bewegliche Ladeeinrichtung (3) eine um eine Mittelachse (B) drehbare Trommel ist.

3. Vorrichtung nach Anspruch 1 oder 2, dadurch gekennzeichnet, dass die Rücklaufsicherungseinrichtungen von mindestens zwei Sperrklinken (31) gebildet sind, die dazu bestimmt sind, mit auf dem Außenumfang des Gehäuses (1) und/oder des Deckels (2) ausgesparten Knaggen (13, 23) zusammenzuwirken.

4. Vorrichtung nach einem der vorstehenden Ansprüche, dadurch gekennzeichnet, dass die Blätter (41, 42) des Gehäuses (1) und des Deckels (2) zu beiden Seiten der Zellen (30) angeordnet sind und eine zentrale Öffnung (41a, 42a) umfassen, deren Durchmesser geringfügig über dem der Gelatinekapseln (G) liegt und die mit dem Ansatzstück (11) und der Lufteinlassöffnung (21) in der Inhalationsposition in Verbindung steht.

5. Vorrichtung nach Anspruch 2, dadurch gekennzeichnet, dass der Deckel (2) des Gehäuses (1) zylindrisch ist und eine Drehachse aufweist, die mit der Mittelachse (B) der Ladeeinrichtung (3) zusammenfällt.

6. Vorrichtung nach einem der vorstehenden Ansprüche, dadurch gekennzeichnet, dass die Ladeeinrichtung (3) und der Deckel (2) durch ein Mitnahmesystem mit Freilauf verbunden sind.

7. Vorrichtung nach einem der vorstehenden Ansprüche, dadurch gekennzeichnet, dass der Deckel (2) mindestens einen peripheren Zapfen (20) umfasst, der verschiebbar in eine unterbrochene Nut (12) eingreift, die auf dem Außenumfang des Gehäuses (1) ausgespart ist und gegen deren Enden der Zapfen (20) anschlägt, um den Gang des Deckels (2) bezüglich des Gehäuses (1) zu begrenzen.

8. Vorrichtung nach einem der vorstehenden Ansprüche, dadurch gekennzeichnet, dass das Ansatzstück (11) eine innere Ableitung (11a) umfasst, die divergent ist und durch eine Klappe (5) verschließbar ist.

9. Vorrichtung nach Anspruch 3, dadurch gekennzeichnet, dass die Sperrklinken (31) der Ladeeinrichtung (3) von Klauen (31a) gebildet sind, die sich im Wesentlichen tangential zur Peripherie der Ladeeinrichtung (3) erstrecken und die sich beim Kontakt mit den Knaggen (13, 23) elastisch biegen können, um sich einzuziehen.

10. Vorrichtung nach den Ansprüchen 3 und 9, dadurch gekennzeichnet, dass sich das freie Ende der Klauen (31a) der Sperrklinken (31) auf dem Halbmesser der Trommel (3) befindet, die die Mitte einer Zelle (30) passiert.

11. Vorrichtung nach Anspruch 7, dadurch gekennzeichnet, dass die Länge der peripheren Nut (12) des Gehäuses (1) dem Abstand entspricht, der zwei Zellen (30) der Ladeeinrichtung (3) trennt.

12. Vorrichtung nach einem der vorstehenden Ansprüche, dadurch gekennzeichnet, dass die Lufteinlassöffnung (21) quer durch den Deckel (2) ausgespart ist und mit einem Schutzgitter (24) versehen ist.

13. Vorrichtung nach einem der vorstehenden Ansprüche, dadurch gekennzeichnet, dass das Gehäuse (1) eine Schale (10) umfasst, in der sich die Ladeeinrichtung (3) befindet.

14. Vorrichtung nach den Ansprüchen 2 und 13, dadurch gekennzeichnet, dass die Achse der Schale mit der Drehmittelachse (B) der Trommel (3) zusammenfällt.

15. Vorrichtung nach einem der vorstehenden Ansprüche, dadurch gekennzeichnet, dass das Gehäuse (1) ein Sichtfenster (15) umfasst, das gegenüber einer der Haltestellungen der Zellen (30) der Ladeeinrichtung (3) angeordnet ist, um ein Kontrollfenster für die Füllung der Ladeeinrichtung zu bilden.

16. Vorrichtung nach einem der vorstehenden Ansprüche, dadurch gekennzeichnet, dass die Länge der Zellen (30) der Ladeeinrichtung (3) unter der Länge der Gelatinekapseln (G) liegt.

17. Vorrichtung nach einem der vorstehenden Ansprüche, dadurch gekennzeichnet, dass die Schneidkanten (41b, 42b) der Blätter (41, 42) des Gehäuses (1) und des Deckels (2) so angeordnet sind, dass die Gelatinekapseln (G) tangential zu ihrer zylindrischen Seitenwand durchschneidbar sind.

18. Vorrichtung nach den Ansprüchen 2 und 3, dadurch gekennzeichnet, dass die Ladeeinrichtung (3) sieben gemäß einem Kreis angeordnete Zellen (30) und sieben entsprechende periphere Sperrklinken (31) einschließt.
